# EUROPÄISCHE PATENTSCHRIFT

(11) **EP 0 522 584 B1**
(45) Veröffentlichungstag und Bekanntmachung des Hinweises auf die Patenterteilung: **27.12.1996**
(21) Anmeldenummer: 92111793.3
(22) Anmeldetag: 10.07.1992
(51) Int. Cl.: A61F 13/15

(54) **Wegwerfhöschen und Verfahren zur Herstellung desselben**
Disposable panty and method of producing the same
Couche culotte à jeter et procédé pour sa fabrication

(30) Priorität: 10.07.1991 JP 195702/91
(43) Veröffentlichungstag der Anmeldung: 13.01.1993
(73) Patentinhaber: UNI-CHARM CORPORATION, Kawanoe-shi Ehime-ken (JP)
(72) Erfinder: Matsushita, Michiko, Ehime-ken (JP)
(74) Vertreter: Sperling, Rüdiger, Dipl.-Ing.

(56) Entgegenhaltungen:
- EP-A- 0 048 011
- EP-A- 0 405 575
- EP-A- 0 417 766
- FR-A- 2 354 064
- US-A- 4 701 171
- US-A- 4 938 753

## Beschreibung

### Der Erfindung zu Grunde liegender Stand der Technik

Die vorliegende Erfindung betrifft Wegwerfhöschen, beispielsweise in Form von Erziehungshöschen für Babys, Höschenwindeln oder Hygienehöschen sowie ein Verfahren zur Herstellung derselben.

Bei herkömmlichen Wegwerfhöschen, beispielsweise Wegwerf-Erziehungshöschen, ist es bekannt, den gesamten Vorder- und Hinterabschnitt aus elastisch dehnbarem Bahnmaterial, wie z.B. Vliesstoff, herzustellen, oder nur die entgegengesetzten Seitenbereiche des Vorder- und Hinterabschnitts aus derartigem Bahnmaterial herzustellen. In dieser Ausführung offenbart die FR-A-2 354 064 ein Baby-Höschen, das aus einem dehnbaren Mittelteil, das vom Rücken zwischen den Beinen hindurch bis zum Bauch reicht, und undehnbaren Seitenteilen besteht.

Die US-A-4 938 753 offenbart einen Schichtaufbau eines Wegwerfhöschens, das aus elastischen Seitenteilen und einem gerafften, aber unelastischen Mittelteil besteht. Im Bereich einer Hüftöffnung enthält das Mittelteil elastische Elemente, die im wesentlichen den Abstand zwischen den Seitenteilen überbrücken. Somit sind derartige bekannte Höschen jedoch wenigstens entlang ihrer entgegengesetzten Seitenränder elastisch dehnbar, und dadurch kann der Vorderabschnitt unter einer Kraft, die auf ihn bei der Bewegung der Beine des Benutzers ausgeübt wird, oder unter dem Gewicht der im Schrittbereich der Höschen angesammelten Exkremente nach unten verlagert werden. Diese Verlagerung des Vorderabschnitts nach unten führt nicht nur zu einem unschönen Erscheinungsbild beim Tragen, sondern auch zu einer Erweiterung der Beinöffnungen, durch die dann Exkremente austreten können.

### Beschreibung der Erfindung

Es ist die Aufgabe der Erfindung, Höschen unter Verwendung einer elastisch dehnbaren Bahn in der Weise auszubilden, daß die Abwärtsverlagerung des Vorderabschnitts möglichst gering gehalten ist.

Diese Aufgabe wird sowohl mit einem Wegwerfhöschen nach Anspruch 1 als auch durch ein Verfahren nach Anspruch 4 gelöst.

Vorteilhafte und daher bevorzugte Weiterbildungen des Wegwerfhöschens bzw. des Verfahrens zu dessen Herstellung sind in den jeweils abhängigen Ansprüchen angegeben.

Gemäß vorliegender Erfindung wird vorstehend genannte Aufgabe durch Wegwerfhöschen gelöst, die einen Vorderabschnitt, der aus Bahnmaterial, das wenigstens in Querrichtung elastisch dehnbar ist, gefertigt ist, und einen Hinterabschnitt, der aus Bahnmaterial gefertigt ist, das im wesentlichen keine elastische Dehnbarkeit aufweist, umfassen, die miteinander verbunden werden, wobei eine Querabmessung des Vorderabschnitts kleiner ist als die Querabmessung des Hinterabschnittes, so daß die seitlich entgegengesetzten Hüftbereiche des Trägers der Höschen von einem Bereich des Hinterabschnitts abgedeckt werden können. Falls die Höschen beispielsweise als Erziehungshöschen oder Höschenwindeln Exkremente, z.B. Urin, absorbieren und aufnehmen sollen, so kann für äußere Lagen des Vorder- bzw. Hinterabschnittes flüssigkeitsundurchlässiges Bahnmaterial verwendet werden, während eine einzelne, nicht verbundene, durchgehende flüssigkeitsdurchlässige Lage, die im wesentlichen nicht elastisch dehnbar ist, als innere Lage verwendet werden kann, die dem Vorder- und Hinterabschnitt gemeinsam ist. Zwischen diesen inneren und den äußeren Lagen kann, falls erwünscht, ein flüssigkeitsabsorbierender Kern liegen.

Während gemäß vorliegender Erfindung auf Wunsch elastisch dehnbare Elemente um die Hüftöffnung und die Beinöffnungen der Höschen zum Verengen dieser Bereiche vorgesehen sein können, ist die Verwendung derartiger elastisch dehnbarer Elemente für die Erfindung nicht von wesentlicher Bedeutung.

Hat ein Benutzer die erfindungsgemäßen Höschen angelegt, so sind die einander seitlich entgegengesetzten Seiten der Hüfte des Benutzers von einem Teil des Hinterabschnittes bedeckt, der im wesentlichen keine elastische Dehnbarkeit aufweist, und die in Querrichtung wirkende kontrahierende Kraft des Vorderabschnittes, der sich zwischen diesen seitlich entgegengesetzten Seiten der Hüfte des Benutzers erstreckt und in wenigstens der Querrichtung über elastische Dehnbarkeit verfügt, verursacht die Anpassung des gesamten Höschens an den Körper des Benutzers. Die einander seitlich entgegengesetzten Seitenbereiche wirken als Mittel, um das Höschen am Körper des Benutzers zu halten. Der am Bauch des Benutzers angeordnete Vorderabschnitt kann sich beim Ausatmen des Benutzers frei zusammenziehen.

### Kurze Beschreibung der Zeichnung:

Die gemäß vorliegender Erfindung aufgebauten Höschen und das Verfahren zur Herstellung derselben werden anhand eines Beispiels unter Bezug auf die beiliegenden Figuren näher erläutert. Es zeigen:
- Fig. 1: eine perspektivische Darstellung eines Höschen, das gemäß einer ersten Ausführungsform der vorliegenden Erfindung aufgebaut ist;
- Fig. 2: eine Teilaufsicht zur Darstellung einiger Schritte, die bei der Herstellung der ersten Ausführungsform durchgeführt werden, d.h. das Verbinden einer Bahn für den Vorderabschnitt und einer Bahn für den Hinterabschnitt zur Bildung einer Bahn für die äußere Lage der Höschen, das Versehen dieser Bahn für die äußere Lage mit elastisch dehnbaren Elementen um eine Hüftöffnung und um Beinöffnungen, das Auflegen einer Bahn für die innere Lage der Höschen auf die Bahn für die äußere Lage der Höschen und das Bilden der Beinöffnungen;
- Fig. 3: eine Teilaufsicht auf die in Fig. 2 zusammengesetzten Bahnen, die entlang einer in Längsrichtung verlaufenden Mittellinie gefaltet wurden und an den gewünschten Stellen mit Siegellinien für die entgegengesetzten Seiten der einzelnen Höschen versehen wurden;
- Fig. 4: eine Draufsicht auf ein durch das Schneiden der in Fig. 3 gezeigten Bahn erhaltenes einzelnes Höschen;
- Fig. 5: eine Teilaufsicht zur Erläuterung einiger Schritte, die bei der Durchführung einer zweiten Ausführungsform des erfindungsgemäßen Verfahrens durchgeführt werden, d.h. das Verbinden der Bahn für den Vorderabschnitt und der Bahn für den Hinterabschnitt miteinander, um die für die äußere Lage der Höschen vorgesehene Bahn zu bilden, und das Versehen der Bahn für die äußere Lage mit elastisch dehnbaren Elementen und weiter mit einem absorbierenden Kern;
- Fig. 6: eine Teilaufsicht zur Erläuterung eines Verfahrensschrittes der zweiten Ausführungsform, d.h. das Versehen der Bahn für die Innenlage der Höschen mit elastisch dehnbaren Elementen um die Hüfte;
- Fig. 7: eine Teilaufsicht auf die in Fig. 6 gezeigte Bahn, die auf die in Fig. 5 gezeigte Bahn aufgelegt ist;
- Fig. 8: eine Teilaufsicht auf die in Fig. 7 gezeigte Bahn, die entlang einer in Längsrichtung verlaufenden Mittellinie zusammengefaltet und mit Beinöffnungen versehen ist; und
- Fig. 9: eine Kombination einer Teilaufsicht der in Fig. 8 gezeigten Bahn, die an gewünschten Stellen mit Siegellinien zur Begrenzung der seitlich entgegengesetzten Ränder der Höschen versehen ist, und einer Aufsicht auf die Bahn nach dem Schneiden zum Erhalten einzelner Höschen.

### Bevorzugte Ausführungsformen der Erfindung

### Ausführungsform des Höschens

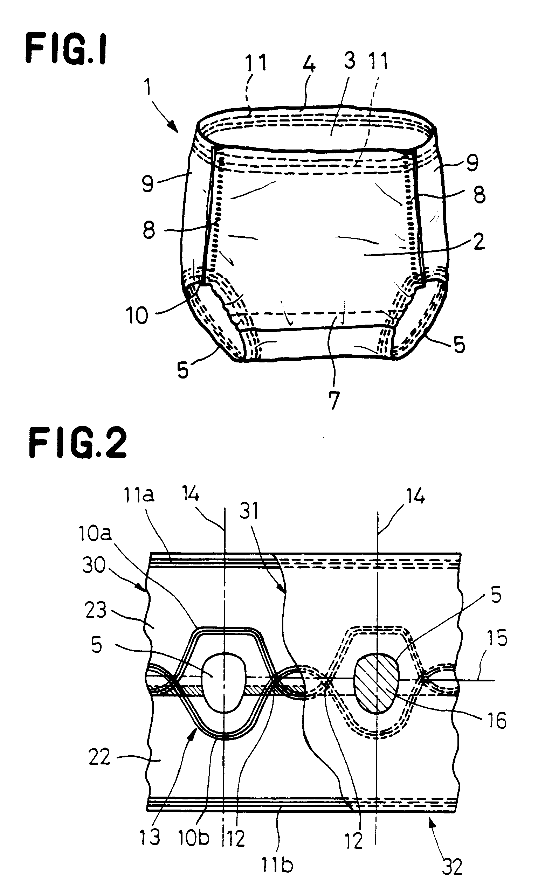

Fig. 1 zeigt die einfachste Ausführung eines gemäß dem erfindungsgemäßen Verfahren aufgebauten Höschens 1.

Das Höschen 1 umfaßt eine Vorderabschnittslage 2, die wenigstens in Querrichtung oder sowohl in der Länge als auch in der Breite elastisch dehnbar ist, und eine Hinterabschnittslage 3, die im wesentlichen nicht elastisch dehnbar ist. Die Höschen haben eine Hüftöffnung 4 und zwei Beinöffnungen 5. In Fig. 1 ist die Vorderabschittslage 2 in Querrichtung kontrahiert dargestellt. In diesem Zustand ist ihre Abmessung in Querrichtung kleiner als die der Hinterabschnittslage 3. Die Vorderabschnittslage 2 ist auch in ihrer Längsabmessung kleiner als die Hinterabschnittslage 3. In Längsrichtung liegende Enden der Vorderabschnitts- und Hinterabschnittslage 2, 3 überlappen einander und sind durch eine kontinuierliche Siegellinie 7 verbunden, die entlang diesem überlappten Bereich verläuft. Die jeweils in Querrichtung einander entgegengesetzten Seitenränder der Vorderabschnitts- und Hinterabschnittslagen 2, 3 überlappen einander an ihren Innenflächen und sind jeweils durch unterbrochene Siegellinien 8, die entlang dieser überlappten Bereiche verlaufen, miteinander verbunden.

Bei in Querrichtung zusammengezogener Vorderabschnittslage 2, wie in der Fig. 1 gezeigt ist, werden einander seitlich entgegengesetzte Seitenabschnitte 9 des Höschens 1 von Teilen der Hinterabschnittslage 3 gebildet und die Verbingdungsbereiche (Siegellinie 8) der seitlich entgegengesetzten Seitenabschnitte 9 liegen im Bauchbereich. Selbstverständlich wird die Vorderabschnittslage 2 in seitlicher Richtung gedehnt und zieht sich anschließend wieder zusammen, wenn die Höschen 1 dem Benutzer angelegt werden. Das Verhältnis der Abmessungen in Querrichtung der Vorderabschnittslage 2 und der Hinterabschnittslage 3, wie auch die Dehnungsrate in Querrichtung der Vorderabschnittslage 2 sind so ausgewählt, daß die Siegellinien 8 wie auch die seitlich entgegengesetzten Seitenabschnitte 9 nach dem Anlegen des Höschens an den Benutzer wie vorstehend erwähnt angeordnet sein können.

Elastisch dehnbare Elemente 10, 11, die jeweils mehrere fadenartige elastische Einzelelemente umfassen, werden in in Längsrichtung gedehntem Zustand um die beiden Öffnungen 5 bzw. die Hüftöffnung 4 angebracht.

Eine hier nicht dargestellte kontinuierliche innere Lage, die dem Vorder- und Hinterabschnitt gemeinsam ist und im wesentlichen keine elastische Dehnbarkeit aufweist, kann, falls erwünscht, in gewünschten Intervallen punktweise mit der Innenseite der Vorderabschnittslage 2 und der Hinterabschnittslage 3 verbunden sein, so daß sie sich nicht störend auf die elastische Dehnbarkeit der Vorderabschnittslage 2 auswirkt. In diesem Fall wird eine flüssigkeitsundurchlässige Bahn als äußere Lage verwendet, während als innere Lage eine entgegengesetzten keitsdurchlässige Bahn verwendet wird, und die seitlich entgegengesetzten Seiten der inneren Bahn werden mit den seitlich entgegengesetzten Enden der Vorderabschnittslage 2 und der Hinterabschnittslage 3 in der vorstehend erwähnten Weise verbunden. In diesem Fall kann auch ein flüssigkeitsabsorbierender Kern zwischen die innere und äußere Lage gelegt sein, falls dies erwünscht ist.

### Erste Ausführungsform des Verfahrens zur Herstellung der Höschen

In Fig. 2 bis 4 sind die jeweiligen Schritte des Verfahrens gemäß vorliegender Erfindung dargestellt. Eine Vorderabschnittsbahn 22, die wenigstens in Querrichtung elastische Dehnbarkeit aufweist, wird einer nicht dargestellten Zusammenbaustation zugeführt, wobei die Bahn 22 mit einer gewünschten Dehnungsrate auf, z.B. das 1,05- bis das 1,5-fache in Längsrichtung gedehnt ist, während eine Hinterabschnittsbahn 23, deren Breite in geeigneter Weise größer ist als die der Bahn 23 und die im wesentlichen keine elastische Dehnbarkeit aufweist, in der Weise der Zusammenbaustation zugeführt wird, daß eine in Längsrichtung verlaufende Seitenkante der Bahn 23 eine in Längsrichtung verlaufende Seitenkante der Bahn 22, die gedehnt vorliegt, überlappt, und dieser überlappte Bereich kann durch die kontinuierliche Siegellinie 7 zusammengefügt werden, um so die die äußere Lage bildende Bahn 30 zu bilden. Das Verbinden oder Zusammenfügen kann durch Verwendung von Heißschmelzkleber oder thermischen oder Ultraschallschweißverfahren erzielt werden.

Die Dehnung der Bahn 22 kann beispielsweise auf eine Weise erreicht werden, wie sie normalerweise zur Dehnung der elastisch dehnbaren Elemente, die um die jeweiligen Beinöffnungen von Wegwerfwindeln oder ähnlichem anzubringen sind, verwendet wird, nämlich durch Erhöhen der Umfangsgeschwindigkeit eines Paares von Klemmwalzen, die in Laufrichtung der Bahn 22 vorgeordnet sind und diese zwischen sich einklemmen, relativ zur Umfangsgeschwindigkeit eines weiteren Paares ähnlicher Klemmwalzen, die in Laufrichtung der Bahn 22 nachgeordnet sind.

Zwei endlose elastisch dehnbare Elemente 10a, b, die jeweils mehrere fadenartige elastische Einzelelemente umfassen, werden entlang des Mittelbereiches der auf die Zusammenbaustation zulaufenden Bahn 30 gelegt und an dieser angebracht, während die elastischen Elemente 10a, 10b in Längsrichtung mit einer gewünschten Dehnungsrate, beispielsweise das ein- bis dreifache gedehnt werden. Die jeweiligen Elemente 10a, 10b bilden dabei einander teilweise überschneidende sinusähnliche Kurven. Gleichzeitig werden zwei weitere endlose, elastisch dehnbare Elemente 11a, 11b, die ebenfalls jeweils mehrere elastische Einzelelemente umfassen, entlang den an die in Längsrichtung entgegengesetzten Seitenränder angrenzenden Bereichen aufgelegt und an der Bahn 30 befestigt.

Das Anordnen der elastisch dehnbaren Elemente 10a, 10b, 11a, 11b in vorstehend beschriebener Konfiguration kann, wie von der Anmelderin vorliegender Patentanmeldung in der europäischen Patentanmeldung Nr. 040 55 75 A1 aufgezeigt, durch Verwendung von zwei Traversiereinrichtungen erreicht werden. Das Befestigen der elastisch dehnbaren Elemente an der Bahn 30 kann durch vorheriges Auftragen von Heißschmelzkleber auf die Bahn 30 entlang der von den jeweiligen elastisch dehnbaren Elementen zu beschreibenden Ortskurven oder durch Auftragen eines derartigen Klebers unmittelbar auf diese elastisch dehnbaren Elemente erzielt werden. Die Abschnitte der jeweiligen elastisch dehnbaren Elemente 10a, 10b, die zwischen zwei benachbarten Kreuzungspunkten derselben liegen, können frei von Klebstoff sein, so daß diese Abschnitte nicht an der Bahn 30 befestigt werden, und darüber hinaus kann jeder dieser Bereiche an einer Stelle durchtrennt werden, so daß diese Abschnitte zurückschnellen.

Eine innere Lagenbahn 31, die im wesentlichen dieselbe Breite wie die auf die Zusammenbaustation zulaufende Bahn 30 aufweist und die im wesentlichen nicht elastisch dehnbar ist, wird auf die Bahn 30 aufgelegt und mit dieser verbunden, um so eine Bahn 32 zu bilden. Derartiges Verbinden kann ebenfalls durch die Verwendung von Heißschmelzkleber oder durch thermische oder Ultraschallschweißverfahren durchgeführt werden.

Aus jeweiligen Kreisbereichen 13, die von den elastisch dehnbaren Elementen 10a, 10b gebildet werden, wird ein Abschnitt 16 in gewünschter Größe und Form, die symmetrisch bezüglich einer imaginären Mittellinie 14 ist, die quer zur Laufrichtung der Bahn 32 über diese verläuft, zur Herstellung der Beinöffnung 5 ausgeschnitten.

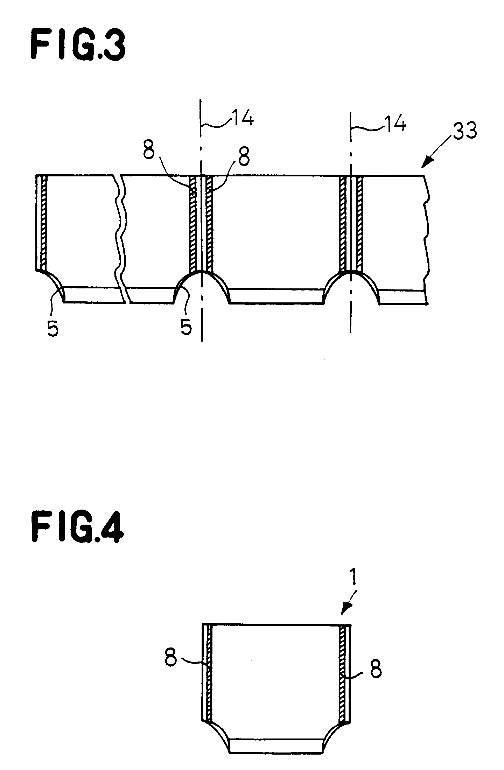

Die so mit Beinöffnungen 5 versehene Bahn 32 wird entlang einer in Längsrichtung verlaufenden imaginären Mittellinie 15 übereinandergefaltet, wobei die Bahn 31 nach innen weist, und mit den Zwischensiegellinien 8 versehen, die unmittelbar an den imaginären Mittellinien 14 verlaufen, wodurch die Bahn 31 und die Bahn 32 miteinander verbunden werden, um so eine Bahn 33 zu bilden (Fig. 3).

Die auf die Zusammenbaustation zulaufende Bahn 33 wird zusammen mit den elastisch dehnbaren Elementen 10a, 10b, 11a, 11b sukzessive entlang den jeweiligen imaginären Mittellinien 14, die jeweils parallel zu und zwischen je zwei Siegellinien verlaufen, durchtrennt, wodurch die einzelnen Höschen erhalten werden.

Die einzelnen Beinöffnungen 5 können auch, anstatt daß sie unmittelbar nach der Herstellung der Bahn 32 gebildet werden, unmittelbar nach der Bildung der Bahn 33 oder während des Trennvorganges der Bahn 33 in Einzelhöschen gebildet werden.

Die äußere Lagenbahn 30 kann aus flüssigkeitsundurchlässigem Material wie etwa Kunststoffolie bestehen, und insbesondere die Bahn 23, die einen Teil der Bahn 30 bildet, kann aus feuchtigkeits- und luftdurchlässigem Material hergestellt sein. Die Innenlagenbahn 31 kann aus Faservliesstoff bestehen. Es sei angemerkt, daß, wie in Bezug auf Fig. 1 beschrieben wurde, das Höschen in seiner einfachsten Konfiguration keine innere Lagenbahn 31 aufweist, das heißt, daß nur die Bahn 30 als Material für die Einzellagen des Höschens verwendet wird. In diesem Fall bestehen die Bahnen 22, 23, die gemeinsam die Bahn 30 bilden, vorzugsweise aus Vliesstoff. Während die elastisch dehnbaren Elemente 10, 11 aus Naturkautschuk oder synthetischem Gummi oder ähnlichem bestehen können, werden diese elastisch dehnbaren Elemente in der einfachsten Ausführung des Höschens 1 manchmal weggelassen.

### Zweite Ausführungsform des Verfahrens zur Herstellung der Höschen

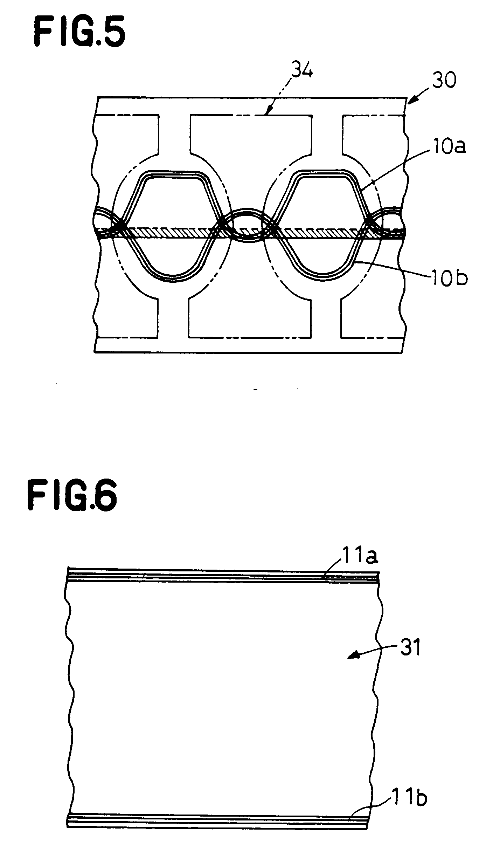

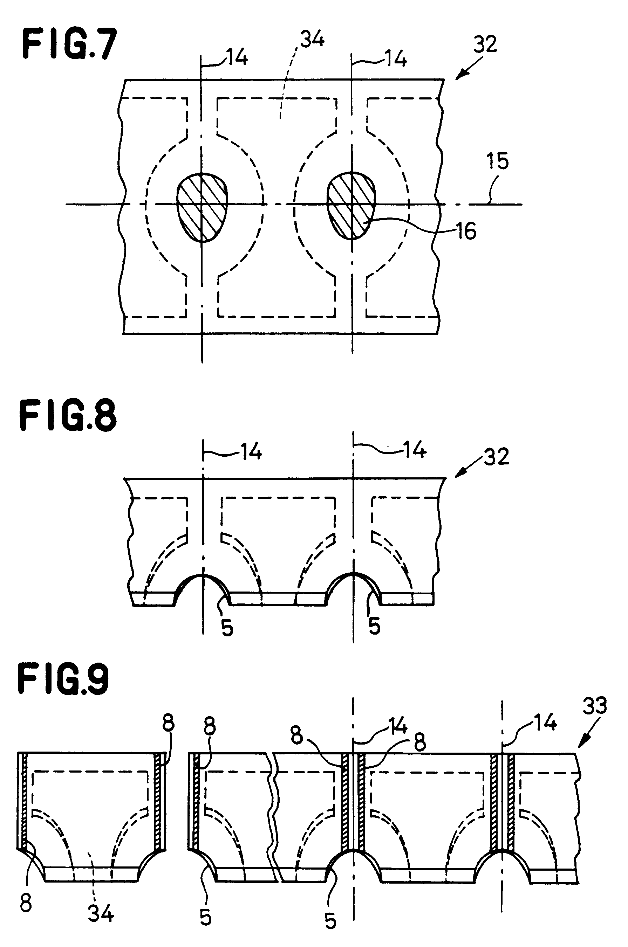

Diese zweite Ausführungsform ist der ersten Ausführungsform ähnlich. Anders als bei dieser wird jedoch hier, wie in Fig. 5 durch unterbrochene Linien dargestellt ist, ein sanduhrförmiger, flüssigkeitsabsorbierender Kern 34 zwischen jeweils zwei Kreisbereichen 13, die von den elastisch dehnbaren Elementen 10a, 10b gebildet sind, eingelegt, bevor die Bahn 31 auf die Bahn 30 aufgelegt wird. Die elastisch dehnbaren Elemente 11a, 11b werden, wie in Fig. 6 dargestellt ist, auf die Innenfläche der Bahn 31 aufgelegt und mit dieser verklebt, und die Beinöffnungen 5 werden, wie aus Fig. 7 bis 9 ersichtlich ist, durch Ausschneiden der entsprechenden Abschnitte aus der Bahn 32 nach dem Falten entlang der in Längsrichtung der Bahn verlaufenden imaginären Mittellinie 15 ausgeschnitten. Daher sind die den Teilen der ersten Ausführungsform entsprechenden Teile mit denselben Bezugszeichen bezeichnet und auf eine weitere detaillierte Beschreibung der zweiten Ausführungsform wird verzichtet. Der Kern 34 umfaßt vorzugsweise zerkleinerte Faserpulpe, gemischt mit thermoplastischen Fasern und hochabsorbierendem Polymerpulver, das in eine gewünschte Form gepreßt ist.

Wenn die erfindungsgemäßen Höschen dem Benutzer angelegt werden, sind die entgegengesetzten Hüftseiten des Körpers des Benutzers von Teilen des Hinterabschnittes bedeckt, der im wesentlichen keine elastische Dehnbarkeit aufweist, und eine in Querrichtung wirkende kontrahierende Kraft des Vorderabschnittes, der zwischen diesen beiden entgegengesetzten Hüftseiten liegt und wenigstens in Querrichtung elastisch dehnbar ist, sorgt dafür, daß das gesamte Höschen passend am Körper des Benutzers anliegt. Damit dienen diese einander entgegengesetzten Hüftseitenabschnitte als Mittel, um das Höschen am Körper des Benutzers zu halten und dadurch den Nachteil zu vermeiden, der herkömmlicherweise aufgrund einer unstabilen Dehnbarkeit dieser entgegengesetzten Hüftseitenabschnitte auftritt, nämlich, daß der Vorderabschnitt unter einer durch die Beinbewegung des Benutzers verursachten Kraft oder unter dem Gewicht von im Schrittbereich der Höschen angesammelten Exkrementen nach unten verlagert wird, was nicht nur zu einem unschönen Erscheinungsbild beim Tragen, sondern auch zu erweiterten Beinöffnungen führt, durch die Exkremente austreten können.

Die derart vorteilhaft ausgebildeten Höschen können ohne weiteres durch das erfindungsgemäße Verfahren hergestellt werden, indem eine in Längsrichtung verlaufende Seitenkante der zweiten Bahn für den Hinterabschnitt, die im wesentlichen keine elastische Dehnbarkeit aufweist, mit einer in Längsrichtung verlaufenden Seitenkante der ersten Bahn für den Vorderabschnitt überlappt wird, die wenigstens in Querrichtung elastisch dehnbar ist, während diese erste Bahn in Längsrichtung gestreckt wird, worauf dieser überlappte Bereich mit einer Siegellinie versehen wird, um die beiden Bahnen miteinander zu verbinden.

## Patentansprüche

1. Wegwerfhöschen (1) mit einer Hüftöffnung (4) und zwei Beinöffnungen (5) sowie einem Vorder- (2) und einem Rückabschnitt (4), wobei der Vorderabschnitt (2) eine wenigstens in Querrichtung elastisch dehnbare Lage (22) umfaßt, seitlich entgegengesetzte Seiten (9) der Vorder- und Hinterabschnitte (2 bzw. 4) mit jeweiligen Siegellinien (8) miteinander verbunden sind, und die Abmessung zumindest der elastisch dehnbaren Lage (22) des Vorderabschnittes (2) in Querrichtung kleiner als die Abmessung des Hinterabschnittes (4) in Querrichtung ist, dadurch gekennzeichnet, daß der Hinterabschnitt (4) eine Lage (23) umfaßt, die im wesentlichen nicht elastisch dehnbar ist, und daß die in Längsrichtung liegenden Enden des Vorder- und Hinterabschnittes (2 bzw. 4) mittels einer Siegellinie (7) in einem Schrittbereich miteinander verbunden sind.

2. Wegwerfhöschen nach Anspruch 1, dadurch gekennzeichnet, daß die wenigstens in Querrichtung elastisch dehnbare Lage (22) des Vorderabschnittes (2) und die im wesentlichen nicht elastisch dehnbare Lage (23) des Hinterabschnittes (4) äußere flüssigkeitsundurchlässige Lagen sind, daß ein Innen-Vorderabschnitt und ein Innen-Hinterabschnitt vorgesehen sind, die eine einzelne, lose, durchgehende flüssigkeitsdurchlässige Bahn (31) enthalten, die im wesentlichen nicht elastisch dehnbar ist und an der Innenseite der äußeren Lagen (22, 23) anliegt, und daß die innere Bahn (31) und die äußeren Lagen (22, 23) ebenfalls entlang ihrer seitlich entgegengesetzten Seitenränder miteinander verbunden sind.

3. Wegwerfhöschen nach Anspruch 2, dadurch gekennzeichnet, daß zwischen der inneren Bahn (31) und den äußeren Lagen (22, 23) ein flüssigkeitsabsorbierender Kern (34) eingelegt ist.

4. Verfahren zur Herstellung von Wegwerfhöschen (1), umfassend die Schritte:
a) Dehnen an einer ersten Bahn (22) für einen Vorderabschnitt (2), der wenigstens in Querrichtung eine elastische Dehnbarkeit aufweist;
b) Überlappen einer in Längsrichtung verlaufenden Seitenkante einer zweiten Bahn (23) für einen Hinterabschitt (4), die im wesentlichen keine elastische Dehnbarkeit aufweist, mit einer in Längsrichtung verlaufende Seitenkante der ersten Bahn (22), während diese in ihrem gedehnten Zustand gehalten ist, worauf dieser überlappte Bereich mit einer ersten Siegellinie (7) versehen wird, entlang der diese Bahnen (22, 23) miteinander verbunden werden, wodurch eine dritte Bahn (30; 32) gebildet wird;
c) Übereinanderfalten der dritten Bahn (30; 32) entlang einer in Längsrichtung verlaufenden imaginären Mittellinie (15) und anschließendes Anbringen von zweiten Siegellinien (8) an dieser gefalteten Bahn (30; 32), die quer zu derselben in Längsrichtung in regelmäßigen Abständen angeordnet sind, um so eine vierte Bahn (33) zu bilden;
d) Durchtrennen der vierten Bahn (33) entlang von imaginären Mittellinien (14) der jeweiligen zweiten Siegellinien (8), um Einzelhöschen (1) zu erhalten, wobei die zweiten Siegellinien (8) teilweise entlang den seitlich gegenüberliegenden Seiten (9) der Einzelhöschen (1) verbleiben; und
e) teilweises Ausschneiden der dritten (30; 32) oder vierten Bahn (33), basierend auf der in Längsrichtung verlaufenden imaginären Mittellinie (15), zur Bildung von Beinöffnungen (5), was unmittelbar nach der Bildung der dritten (30; 32) oder der vierten Bahn (33) oder während des Abtrennens der vierten Bahn (33) in Einzelhöschen (1) erfolgt.

5. Verfahren zur Herstellung von Wegwerfhöschen nach Anspruch 4, dadurch gekennzeichnet, daß die erste Bahn (22) und die zweite Bahn (23) flüssigkeitsundurchlässige Lagen enthalten, und daß nach dem Anbringen der ersten Siegellinie (7) zum Verbinden der ersten und zweiten Bahnen (22, 23) darauf eine Innenbahn (31) aufgelegt wird, die im wesentlichen keine elastische Dehnbarkeit aufweist, um so die dritte Bahn (32) zu bilden.

6. Verfahren zur Herstellung von Wegwerfhöschen nach Anspruch 5, dadurch gekennzeichnet, daß auf die ersten und zweiten Bahnen (22, 23) in den Einzelhöschen (1) jeweils entsprechenden Bereichen flüssigkeitsabsorbierende Kerne (34) gelegt werden, bevor die Innenbahn (31) darauf aufgelegt wird.

## Claims

1. Disposable panties (1), with hip opening (4) and two leg openings (5) and a front section (2) and a rear section (3), wherein the front section (2) comprises a layer (22) which is elastically extensible at least in the transverse direction, laterally opposite sides (9) of the front and rear sections (2 and 3) being connected together by respective seal lines (8) and the dimension of at least the elastically extensible layer (22) of the front section (2) in the transverse direction being smaller than the dimension of the rear section (3) in the transverse direction, characterized in that the rear section (3) comprises a layer (23) which is substantially not elastically extensible and in that the ends of the front and rear sections (2 and 3) lying in the longitudinal direction are joined together by means of a seal line (7) in a crotch region.

2. Disposable panties according to claim 1, characterized in that the layer (22) of the front section (2) which is elastically extensible at least in the transverse direction and the layer (23) of the rear section (3) which is substantially not elastically extensible are outer layers impermeable to liquids, in that inner front and inner rear sections are provided, which comprise a single, loose, continuous band (31) which is permeable to liquids and is substantially not elastically extensible and lies on the inside of the outer layers (22, 23), and in that the inner band (31) and the outer layers (22, 23) are likewise bonded together along their laterally opposite side edges.

3. Disposable panties according to claim 2, characterized in that a liquid absorbent core (34) is placed between the inner band (31) and the outer layers (22, 23).

4. A method of making disposable panties (1), comprising the steps:
a) stretching a first band (22) for a front section (2) which can extend elastically at least in the transverse direction;
b) overlapping a side edge running in the longitudinal direction of a second band (23) for a rear section (3), which has substantially no ability to extend elastically, a side edge of the first band (22) running in the longitudinal direction, while this is held in its extended state, whereafter this overlapped region is provided with a first seal line (7) along which these bands (22, 23) are connected together, whereby a third band (30; 32) is formed;
c) folding the third band (30; 32) over itself along a conceptual centre line running in the longitudinal direction and then forming second seal lines (8) on this folded band (30; 32) which run transverse thereto at regular intervals in the longitudinal direction, in order thus to form a fourth band (33);
d) parting the fourth band (33) along conceptual centre lines (14) of the respective second seal lines (8, in order to obtain individual panties (1), wherein the second seal lines (8) remain in part along the laterally opposite sides (9) of the individual panties (1); and
e) partially cutting out the third (30; 32) or fourth band (33), based on the conceptual centre line (15) running in the longitudinal direction, in order to form leg openings (5), this being effected directly after forming the third (30; 32) or the fourth band (33) or during the parting of the fourth band (33) into individual panties (1).

5. A method of making disposable panties according to claim 4, characterized in that the first band (22) and the second band (23) comprise layers impermeable to liquids and in that, after forming the first seal lines (7) to bond the first and second bands (22, 23), an inner band (31) is laid thereon, having substantially no ability to extend elastically, in order thus to form the third band (32).

6. A method of making disposable panties according to claim 5, characterized in that respective liquid absorbent cores (34) are laid on to the regions of the first and second bands (22,23) corresponding to the individual panties (1), before the inner band (31) is laid thereon.

## Revendications

1. Petite culotte jetable (1), comprenant une ouverture de hanches (4) et deux ouvertures de passage des jambes (5), ainsi qu'une partie avant (2) et une partie arrière (4), et dans laquelle la partie avant (2) comporte une couche (22) élastique étirable au moins dans le sens transversal, des côtés (9) latéralement opposés des parties avant et arrière (2 et 4) sont raccordés entre eux par des lignes de soudure (8), et dans le sens transversal, la dimension d'au moins la couche (22) élastique étirable de la partie avant (2) est plus petite que celle de la partie arrière (4), caractérisée en ce que la partie arrière (4) comprend une couche (23) qui n'est pratiquement pas étirable élastiquement, et en ce que les extrémités longitudinales des parties avant et arrière (2 et 4) sont raccordées entre elles au moyen d'une ligne de soudure (7) dans une zone d'entre-jambe.

2. Petite culotte jetable selon la revendication 1, caractérisée en ce que la couche (22) de la partie avant (2), élastiquement étirable dans le sens transversal, et la couche (23) de la partie arrière (4), pour l'essentiel non-étirable élastiquement, sont des couches extérieures imperméables aux liquides, en ce qu'il est prévu une partie avant intérieure et une partie avant extérieure, lesquelles contiennent une feuille (31) individuelle, flottante, allant de bout en bout et perméable aux liquides, qui est pour l'essentiel non-étirable élastiquement, et est en contact avec la face intérieure des couches extérieures (22, 23), et en ce que la feuille intérieure (31) et les couches extérieures (22, 23) sont également raccordées entre elles par leurs bords latéraux en opposition latérale mutuelle.

3. Petite culotte jetable selon la revendication 2, caractérisée en ce qu'un corps central absorbant les liquides (34) est inséré entre la feuille intérieure (31) et les couches extérieures (22, 23).

4. Procédé de fabrication de petites culottes jetables (1), comprenant les opérations consistant :
a) à étirer une première bande (22) pour une partie avant (2), qui présente une propension à l'étirage élastique, au moins transversalement;
b) à placer en chevauchement un bord latéral longitudinal d'une deuxième bande (23) destinée à une partie arrière (4), qui ne présente autant dire pas d'aptitude à l'étirage élastique, et un bord latéral longitudinal de ladite première bande (22), pendant que cette dernière est maintenue à l'état étiré, après quoi cette zone en chevauchement est dotée d'une première ligne de soudure (7), suivant laquelle ces bandes (22, 23) sont jointes entre elles, ce qui donne naissance à une troisième bande (30; 32);
c) à plier cette troisième bande (30; 32) sur elle même, en suivant une ligne médiane imaginaire (15) la traversant dans la longueur, et à appliquer ensuite sur cette bande repliée (30; 32) des secondes lignes de soudure (8) disposées à intervalles réguliers, en travers de cette dernière, dans le sens longitudinal, pour former ainsi une quatrième bande (33);
d) à sectionner la quatrième bande (33) en suivant des lignes médianes imaginaires (14) des secondes lignes de soudure (8) respectives, de façon à obtenir des petites culottes individuelles (1), opération à l'issue de laquelle les secondes lignes de soudure (8) sont en partie conservées le long des côtés (9) latéralement opposés des petites culottes (1); et
e) à découper, partiellement, la troisième (30; 32) ou quatrième (33) bande, en prenant pour repère la ligne médiane imaginaire longitudinale (15), pour former des ouvertures (5) de passage des jambes, ce qui se fait immédiatement après la formation de la troisième (30; 32) ou quatrième (33) bande, ou pendant la division de la quatrième bande (33) en petites culottes individuelles (1).

5. Procédé de fabrication de petites culottes jetables selon la revendication 4, caractérisé en ce que la première bande (22) et la deuxième bande (23) renferment des couches imperméables aux liquides, et en ce qu'après l'application de la première ligne de soudure (7) destinée à raccorder les première et deuxième bandes (22, 23), on y dépose une feuille continue intérieure (31), qui n'est pratiquement pas étirable élastiquement, pour former ainsi la troisième bande (32).

6. Procédé de fabrication de petites culottes jetables selon la revendication 5, caractérisé en ce qu'on dépose une couche (34) absorbant les liquides sur les première et deuxième bandes (22, 23), dans des zones correspondant chaque fois aux petites culottes individuelles (1), avant d'y poser la bande intérieure (31).
